Europäisches Patentamt

**European Patent Office**

Office européen des brevets

(19)

(11) **EP 1 269 993 A1**

(12) **EUROPEAN PATENT APPLICATION**

(43) Date of publication:
**02.01.2003 Bulletin 2003/01**

(51) Int Cl.7: **A61K 9/127**

(21) Application number: **01202401.4**

(22) Date of filing: **21.06.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI** | • **Poolman, Berend**<br>**9751 AC Haren (NL)**<br>• **Feringa, Bernard Lucas**<br>**9765 EP Paterswolde (NL)**<br>• **Engberts, Jan Bernard Frederik Nicolaas**<br>**9742 EC Groningen (NL)** |
| (71) Applicant: **Applied NanoSystems B.V.**<br>**9711 BC Groningen (NL)** | (74) Representative: **Prins, Adrianus Willem et al**<br>**Vereenigde,**<br>**Nieuwe Parklaan 97**<br>**2587 BN Den Haag (NL)** |
| (72) Inventors:<br>• **Friesen, Robert Heinz Edward**<br>**9753 CB Haren (NL)** | |

(54) **Delivery of small hydrophilic molecules packaged into lipid vesicles**

(57)    Means and methods are provided for the generation and use of delivery vehicles for small molecules. In one aspect of the invention lipid vesicles comprising proteinaceous channel and said small molecules are generated. Said proteinaceous channel and/or said lipid vesicle are formulated such that said small molecule is released in the vicinity of a target cell. Said target cell may be present in vitro or in vivo.

EP 1 269 993 A1

Printed by Jouve, 75001 PARIS (FR)

## Description

The invention relates to the field of medicine. More in particular the invention relates to the field of pharmacology.

Liposomes are typically spherical lipid bilayers from 50 nm to 1000 nm in diameter, and serve as convenient delivery vehicles for biologically active molecules. Lipid/drug aggregates are easy to form and vulnerable to structural manipulations, allowing for the adjustment of their properties for particular purposes. In selected cases, the application of liposomes in pharmacological therapy improves drug pharmacokinetics compared to its free form. The major advantages of the liposome application are: the protection of active compounds from degradation; the increase in circulation time and the possibility to achieve partial or total tissue or cell selectivity. Selectivity alone improves drug potency, eliminates side effects and allows for dosage reduction.

Although liposome mediated delivery of biologically active molecules is a very promising approach, there are also limitations associated with the current forms. A particular problem is the hydrophobic nature of the lipid bilayer. Hydrophilic drugs are typically not easily released from liposomes. In order to effectively release hydrophilic compounds, the integrity of the bilayer needs to be disrupted. Very often this is not possible in a controlled fashion.

The present invention provides a method for obtaining controlled release of hydrophilic drugs from liposomes. To this end, the present invention in one aspect provides a method for delivering a small hydrophilic molecule to a cell, comprising loading a lipid vesicle with said small molecule and administering said vesicle to fluid that is in contact with said cell, said vesicle further comprising a proteinaceous channel, said channel in the open state allowing passage of said small molecule to the exterior of said vesicle in the vicinity of said cell. Said vesicles can be administered to culture medium of cells growing in vitro. However, preferably, said vesicle is administered to an animal or a human. More preferably, said vesicle is administered to a human. The proteinaceous channel can be any proteinaceous channel that allows passage of said small molecule. Preferably, said proteinaceous channel comprises a solute channel. A solute channel is capable of allowing passage of ions and small hydrophilic molecules. Preferably, said proteinaceous channel comprises an ion channel. More preferably, said proteinaceous channel comprises a mechanosensitive channel. Preferably, a mechanosensitive channel of large conductance (MscL) or a functional equivalent thereof. The invention further provides a method for delivering a small hydrophilic molecule to a cell, comprising loading a lipid vesicle with said small molecule and administering said vesicle to an individual comprising said cell, said vesicle further comprising a mechanosensitive channel of large conductance (MscL) or a functional equivalent thereof, said channel in the open state allowing passage of said small molecule to the exterior of said vesicle. In nature MscL allows bacteria to rapidly adapt to a sudden change in environmental conditions such as osmolarity. The MscL channel opens in response to increases in membrane tension, which allows for the efflux of cytoplasmic constituents. By allowing passage of said constituents to the outside of the prokaryote, it is able to reduce the damage that the sudden change in environmental conditions would have otherwise inflicted. The genes encoding MscL homologues from various prokaryotes are cloned [8]. Nucleic acid and amino acid sequences are available and have been used to obtain heterologous (over)-expression of several MscL [8]. In the present invention lipid vesicles, preferably liposomes, comprising MscL or a functional equivalent thereof are loaded with small hydrophilic molecules whereupon loaded small molecules can be released from said vesicles under activation or opening of the channel. Loading of the lipid vesicle can be accomplished in many ways as long as the small molecules are dissolved in a hydrophilic solvent which is separated from the surrounding hydrophilic solvent by a lipid bilayer.

An advantage of a method of the invention is that the lipid vesicle can be formulated to allow preferential opening of said channel near cells of a selected tissue. Activation of MscL has been found to be controllable. It is possible to tune the type and relative amount of lipids in the vesicle such that the amount of membrane tension required to activate the channel is altered. Thus depending on the circumstances near cells of selected tissue, the lipid vesicle can be tuned to allow preferential activation of the channel and thus preferential release of said small molecule in the vicinity of said cells of said tissue.

Release near a cell or in the vicinity of cells is obtained when release is in fluid that is in contact with said cell. For instance, release into culture medium cells is for the present intended to mean release near or in the vicinity of said cells. The terms "near" or "vicinity" as used herein typically refer to a functional distance rather than a physical distance. For instance, release of a small molecule from a lipid vesicle of the invention in a capillary vessel that feeds target cells is release "near" or in the "vicinity" of said target cells. In contrast, release of said small molecule in blood-vessels that carry blood away from said target cells can be as close physically as said in the feeding vessels but are for the present invention not considered to be release near or in the vicinity of said target cells, because in this case the released small molecule is carried away from the target cells. An exception is made for lymphe and similar fluid, although these types of fluid are carried away from target cells, the contact with the surrounding cells is so intense that said small molecule can still exert effect upon release. If a physical distance is used to define the terms "near" or "in the vicinity of" said physical distance is typically not more than 100 times and preferably not more than 20 times the radius of a target cell, morepreferably, said distance is not more than 10 times the radius of a target cell.

Compositions comprising lipid vesicles have been used in vivo, for instance to enable delivery of nucleic acid or anti-tumour drugs to cells. It has been observed that blood stream administration of such vesicles often leads to uptake of vesicles by cells. Uptake by cells seems to correlate with the charge of the lipid in the vesicle. Uptake is particularly a problem with negatively charged lipid vesicles, these vesicles are very quickly removed from the blood stream by the mononuclear phagocytic system (MPS) in the liver and the spleen. Although the present invention may be used to facilitate uptake of small molecules by cells, it is preferred that the small molecules are delivered to the outside of cells. In the present invention it has been found that MscL is also active in lipid vesicles that consist of positively and/ or neutrally charged lipids. Lipid vesicles comprising said positively and/or neutrally charged lipids are more resistant to uptake by cells of the MPS. Lipid vesicles of the invention therefore preferably comprise positively and/or neutrally charged lipids. Such vesicles exhibit improved half-lifes in the bloodstream. Such vesicles demonstrate improved targeting to non-MPS cells. The lipid part of a lipid vesicle of the invention directed toward the exterior preferably consists predominantly of positively and/or neutrally charged lipids, thereby nearly completely avoiding cellular uptake through negatively charged lipid and thereby further increasing the bloodstream half life of lipid vesicles of the invention. Apart from increasing the half life of the vesicle in the blood stream, positively and/or neutrally charged lipids can also be used to alter the amount of added pressure needed to activate the channel in the vesicle. Vesicles of the invention wherein the outwardly directed lipid part of a lipid vesicle consists predominantly of positively and/or neutrally charged lipids, postpone the rapid cellular uptake as seen for vesicles wherein the outwardly directed part consists of negatively charged lipid. Postponed uptake through the MPS system leads to increased circulation times. Apart from this, positively and/or negatively charged lipids can also be used to alter the amount of membrane tension needed to activate the channel.

The signal or event leading to activation of a channel of the invention can also be changed by altering the MscL in the vesicle. Besides the pH sensitive mutants, other mutants are available that have a higher open probability as compared to the wild type MscL from *Escherichia coli* [11, 14]. This property can be used to tune the activation potential of the channel in a method or composition of the invention. For instance, it is known that in tumours the pH is very often considerably lower than in the normal tissue surrounding the tumour. Other areas in the body that have a lowered pH are the liver, area's of inflammation and ischemic area's. A lower pH can be used as a trigger for activation of the MscL in a vesicle of the invention. Mutant MscL are available that activate (open) in response to a pH that is frequently encountered in tumours. One non-limiting example of such a pH-sensitive mutant is the G22H mutant. This mutant exhibits a higher open probability at (low) pH values that are frequently encountered in tumours, as compared to normal pH values of circulating blood [9]. Thus in a preferred embodiment of a method or composition of the invention, said mutant allows preferred release of said small molecule in said target tissue.

A small molecule can be any hydrophilic molecule small enough to pass through the pore of a channel of the invention. Preferably said small molecule comprises a diameter of no more than 60 Å, more preferably no more than 50 Å and still more preferably no more than 40 Å. Particularly peptides are preferred for the present invention. Peptides typically have very poor pharmacodynamic properties when injected into the bloodstream. With the present invention it is possible to significantly increase the half life of peptides in the circulation. Moreover, by enabling controlled release of a small molecule with a vesicle of the invention it is also possible to have locally a relatively high bioavailability of the peptide, whereas systemically the bioavailability is low or even absent. This also allows for the therapeutic use of molecules that are otherwise too toxic when bioavailable systemically.

Controlled and/or localised release of a small molecule can be achieved in many ways. By, for instance, tuning of the composition of the lipid vesicle and/ or the use of a mutant MscL it is possible to control how and where release of the small molecule will occur. In a preferred embodiment, activation of said channel is triggered upon the availability of a signal. The signal for activation can for instance be exposure of the vesicle to a certain pH, to lightor to a certain temperature. Exposure to the signal can directly or indirectly (through an intermediary signal) lead to the activation of the channel. Preferably, said signal comprises light. There are hydrophobic compounds such as azobenzene phospholipids and related compounds available [10], that mix with the lipids in the vesicle, and that upon exposure to light undergo a structural change such that the gating of the MscL channel can be controlled. It is also possible to insert a photosenstive mutant MscL in the lipid vesicle. Upon exposure to light, a photoreactive molecule conjugated to a specific site of the MscL protein alters conformation thereby controlling the gating of the MscL channel. Activation through light is just one example of an embodiment wherein opening/activation of the channel can be induced by another signal than membrane tension. An alteration in the redox-potential is another non-limiting example. MscL can be made sensitive to the local redox-potential after conjugation of a redox-sensitive molecule, such as a nicotinamide adenine dinucleotide derivative, to a specific site of the MscL protein. Such a redox-sensitive MscL can be (de)activated by changing the redox-potential of the environment. In yet another embodiment said signal comprises an altered pH, preferably said pH is equal or less then 6.5. Recognition of only the open conformation of MscL by an antibody is another non-limiting example of an embodiment that gating of the channel can be induced by another signal than membrane tension. Such an antibody can for instance be used to preferentially increase the open probability of the channel near target cells. A bi-specific antibody comprising the above mentioned specificity for the open state and a specificity for a target cell can be used to accumulate open vesicles near target cells. Another example of a signal that triggers activation of a MscL is local anaesthetics [13]. Local anaesthetics most probably work to activate the channel through their incorporation in the lipid bilayer, which changes the bilayer properties.

There are very likely many substances that can cause activation of the channels. One example to note is this context is [2-(trimethylammonium)ethyl]methanethiosulfonate bromide (MTSES). This compound is capable of associating with MscL mutant G22C. Whereas a hydrophobic moiety at this position makes the channel harder to open, a hydrophilic addition at this position helps to overcome the mechanical work required to open the channel. The compound MTSES helps to lower the amount of signal required to activate the channel (16). Various polar and nonpolar variants of MTSET exist that can be used depending on whether the channel should be easier or more difficult to activate. It is also possible and for some applications even preferred to change the signal needed for activation of the channel from membranepressure to another signal. Signals such as light, local anaesthetics, pH, temperature, etc., can be used to facilitate the local delivery of an incorporated small molecule. For instance, through local illumination a circulating lipid vesicle can be triggered to release incorporated molecules only in the illuminated area of the body. This is an important additional advantage of having another signal or an intermediate signal than pressure for activation of the channel. In a preferred embodiment a vesicle of the invention comprises an asymmetrical bilayer. An asymmetrical bilayer is yet another example of a method to tune the lipid vesicle such that the activation of the channel is altered. It seems that the force gating MscL is from the lipid bilayer and amphipaths probably generate this force by differential insertion into the two leaflets [13]. In a preferred embodiment, a signal required for activation is provided through an intermediate. The intermediate is here capable of transforming the given signal into a pressure signal thereby allowing, if sufficient, the opening of the channel.

In one aspect the invention provides a composition comprising a lipid vesicle comprising a proteinaceous channel and a small hydrophilic molecule, wherein said lipid vesicle and/or said proteinaceous channel is formulated such that said proteinaceous channel is in the open state in the vicinity of a target cell. Preferably, said proteinaceous channel comprises an MscL or functional part, derivative and/or analogue thereof. In a preferred aspect the invention provides a composition comprising a lipid vesicle comprising an MscL or functional part, derivative and/or analogue thereof, wherein said composition is formulated and prepared for use in a human. Preferably said lipid vesicle comprises a small hydrophilic molecule capable of passing through an activated MscL. Preferably, said composition is used in the preparation of a medicament. Preferably said small molecule is intended to be delivered to the outside of a cell in said tissue. A composition as described is of course ideally suited to be used in a method of the invention. Preferably, said MscL is a mutant MscL or a functional part, derivative and/or analogue thereof. A functional part of MscL comprises at least the region that in *E.coli* comprises residue 4 to 110 [11]. It is possible to generate MscL proteins that comprise amino-acid substitution(s), insertion(s) and/or deletion(s) compared to the protein found in bacteria. Such derivatives can of course also be used for the present invention provided that the derivative is functional, i.e. comprises the channel activity in kind, not necessarily in amount. The channel activity may, as will be apparent from the description, be triggerably by means other than pressure. With activity in kind is therefor not meant the type of triggering but the channeling activity, the capability of the protein to allow passage of a hydrophilic substance from one side of the lipid obstruction to the other. The amount of activity, both in the amount of small molecules that may pass per time unit, or the size of the pore through which the small molecule can pass may differ. A derivative of MscL is also an MscL that comprise more or less or different (post-translational) modifications as compared to the native protein. Other options with mutant or derivative channels would be using MscL with genetically engineered changes in the outside loop, like receptor recognising domains (e.g. RGD) that upon binding with the receptor undergo conformation changes that induce opening of the channel. One may also add (part of) an antibody to that loop that induces channel opening after ligand binding. An MscL analogue is a molecule comprising the same activity in kind to allow passage of hydrophilic molecules through a lipid obstruction than MscL itself, not necessarily in amount.

In another aspect the invention provides a method for generating a vehicle for delivery of a small hydrophilic molecule to a cell, said method comprising generating in an aqueous fluid, a lipid vesicle comprising a proteinaceous channel, said vehicle formulated such that said proteinaceous channel is in the open state in the vicinity of said cell. Preferably, said proteinaceous channel assumes said open state upon entering the vicinity of said cell. More preferably, said lipid vesicle further comprises said small molecule. A method for the generation of a vehicle as described above can be advantageously used to generate a composition of the invention.

In a preferred embodiment a lipid vesicle of the invention further comprises a non-channel protein. Preferably, said non-channel protein is a binding molecule capable of binding to a binding partner in said tissue thereby enabling at least a prolonged stay of said vesicle in said tissue and/or near a target cell.

In another aspect the invention provides the use of a lipid vesicle comprising an MscL for controlling delivery of a small hydrophilic molecule to a target tissue in a body.

A lipid vesicle of the invention may be used to deliver a small molecule to any part of the body. However, preferably it is used to deliver to tissue with permeable endothelium such as the liver, the spleen area's of inflammation or tumour bearing tissues.

A lipid vesicle can comprise lipid but may also comprise other molecules. Glycolipids or lipids modified in other ways that maintain the classical bipolarity of a lipid molecule in kind, not necessarily in amount are also called lipids in the present invention. In a preferred embodiment of the invention said lipid vesicle comprises a liposome, more preferably a long circulating liposome. Long circulating liposomes are typically small (150 nm or smaller), neutral and have a specific composition (cholesterol-containing with either phosphatidylcholine and PEG or sphingomyelin etc).

Considering that MscL is typically a foreign protein it is conceivable that upon repeated administration an immune response is mounted by the host. To allow at least a partly evasion of the immune system of the host so called masking groups can be attached to the outside of the lipid vesicle. Preferably, said masking groups comprise PEG.

Non-limiting examples of small molecules that may advantageously be used in a lipid vesicle of the invention are :

- *Interleukins*: peptides and proteins that modulate the immune response
- *Diphteria toxin (fragment):* potent inhibitor of protein synthesis in human cells
- *Muramyl dipeptide*: activator of immune system; macrophage-mediated destruction of tumor cells
- *Cis-4-hydroxyproline*: potential treatment for lung fibrosis
- *Cisplatin (derivatives)*: cancer treatment
- *Cytosine arabinose*: cancer treatment
- *Phosphonopeptides*: antibacterial agent
- β-*Glucuronidase*: activator of prodrugs (e.g., epirubicin-glucuronide)
- *Cytostatic drugs (doxorubicin,ciplatin etc.)*
- *Small therapeutic proteins/peptides (interleukins, growth factors, chemokines)*

MATERIAL AND METHODS

MscL Expression and Purification

*E.coli* PB104 cells containing the plasmid pB104 carrying the MscL-6His construct was grown to mid-logarithmic phase in Luria Bertani medium (10L fermentor) and induced for 4 h with 0.8 mM IPTG [3]. Cells were French-pressed and membranes were isolated by differential centrifugation, as previously described [1]. The membrane pellet (5-8 g wet weight) was solubilized in 100 mL of buffer A (50 mM $Na_2HPO_4.NaH_2PO_4$, 300 mM NaCl, 10 mM imidazole) containing 3% n-octyl β-glucoside. The extract was cleared by centrifugation at 120 000 x g for 35 min, mixed with 4 mL (bed volume) $Ni^{2+}$-NTA agarose beads (Qiagen, Chatworth, CA) equilibrated with buffer A and gently rotated for 15 min (batch loading). The column material was poured into a Bio-Spin column (Bio-Rad) and washed with 10 column volumes of buffer B (as buffer A, except 1% n-octyl β-glucoside) followed by 5 column volumes of the buffer B but with 100 mM imidazole. The protein was eluted with buffer B but with 300 mM imidazole. Eluted protein samples were analysed by fractionation on a SDS-15 % polyacrylamide gel followed by staining with Coomassie Blue or transferring the fractionated proteins to PVDF membranes by semi-dry electrophoretic blotting for immunodetection with a anti-His antibody (Amerham Pharmacia Biotech). Immunodetection was performed with an alkaline phosphatase conjugated secondary antibody as recommended by the manufacturer (Sigma).

Electrospray Ionization Mass Spectrometry of Detergent Solubilized MscL proteins.

Purified detergent solubilized G22C-MscL-6His was heated to 60 °C for 15' and precipitated protein was spun down at 14 000 rpm in a tabletop centrifuge (Eppendorf) for 5 min. The pellet was dissolved in 50% formic acid and 50% acetonitril just before electrospray ionization mass spectrometry (ESI-MS) analysis. The average molecular masses of the proteins were calculated from the m/z peaks in the charge distribution profiles of the multiple charged ions. Spectral deconvolution was performed on the peaks over the mass range from 800 to 1700 using the computer program MacSpec (Sciex). All molecular masses quoted in this paper are average, chemical atomic masses.

2-Sulfonatoethyl methanethiosulfonate Labeling of G22C-MscL-6His.

The single cysteine mutant, G22C-MscL-6His, was labeled with (2-sulfonatoethyl)methanethiosulfonate (MTSES) . A suspension of 20-30 μM of G22C-MscL-6His in buffer B with 300 mM imidazole (0.5 mL final volume), was incubated with 0.6 mM MTSES at 4 °C for 30 min. Conjugation was monitored employing ESI-MS.

Membrane Reconstitution of 6His-MscL

Dry lipid mixtures were prepared by co-dissolving lipids (Avanti Polar Lipids, Alabaster, AL) in chloroform, in weight-fractions as indicated in the experiments, and removing the chloroform by evaporation under vacuum for 4 h. All acyl chains of the synthetic lipids were of the type, dioleoyl, unless indicated otherwise. The dried lipid film was dissolved (20 mg/mL) in 50 mM potassium phosphate, pH 7.0, followed by three freeze/thaw cycles. An aliquot, 200 μL of the rehydrated liposomes and 5% n-octyl β-glucoside, was added to 200 μL purified 6His-MscL. Final protein-to-lipid molar ratio as indicated in the experiments. Subsequent membrane reconstitution was achieved by exhaustive dialysis into a buffer containing 0.1 mM $Na_2HPO_4.NaH_2PO_4$ pH 6.8 containing detergent-absorbing Bio-Beads SM-2 (Bio-Rad, Inc.).

Sucrose Gradient Centrifugation.

Discontinuous sucrose gradients were employed to analyze membrane reconstituted 6His-MscL as described elsewere [6].

Freeze-Fracture Electron Microscopy.

Freeze-fracture electron microscopy of membrane-reconstituted 6His-MscL were performed as described elsewere [7].

Electrophysiologic Characterization of Membrane-Reconstituted MscL.

MscL was reconstituted into liposomes of different lipid composition and aliquots of 200 μL were centrifuged at 48 000 rpm in a tabletop ultracentrifuge (Beckmann). Pelleted proteoliposomes were resuspended into 40 μL buffer C (10 mM 4-morpholinepropanesulfonic acid (MOPS)-buffer, 5% ethylene glycol, pH 7.2), and 20 μL droplets were subjected to dehydration-rehydration cycle on glass slides [2]. Rehydrated proteoliposomes were analysed employing patch-clamp experiments as described previously [3].

In vitro Release Profiles of a Model Drug from Proteoliposomes

The percentage release of a fluorescent model drug, calcein, from MscL-containing liposomes was calculated from the dequenching of calcein fluorescence according to the following equation:

$$\% \text{ Release} = \frac{F_x - F_0}{F_t - F_0} \times 100$$

Where $F_0$ is the fluorescence intensity at zero time incubation, $F_x$ is the fluorescence at the given incubation time-points and $F_t$ is the total fluorescence, obtained after Triton X-100 lysis. Fluoresence was monitored with a SLM 500 spectrofluorimeter in a thermostatted cuvette (1 mL) at 37 °C, under constant stirring. Excitation and emission wavelengths were, respectively, 490 (slit 2 nm) and 520 nm (slit 4 nm). The experiments were performed at lipid concentrations of approximately 50 μM. Control, and MscL-containing liposomes were prepared as described above followed by mixing with an equal volume of 200 mM calcein in PBS buffer. Then a freeze-thaw cycle has been repeated three times followed by extrusion through a 100 nm polycarbonate membrane [12]. The liposomes were separated from free calcein by using Sephadex 50 column chromatography equilibrated with PBS (160 mM NaCl, 3.2 mM KCl, 1.8 mM $KH_2PO_4$, 0.12 mM $Na_2HPO_4$, 1.2 mM EGTA, pH 8.0), which was isotonic to the calcein-containing buffer.

RESULTS AND DISCUSSION

Overexpression and purification of the MscL channel protein.

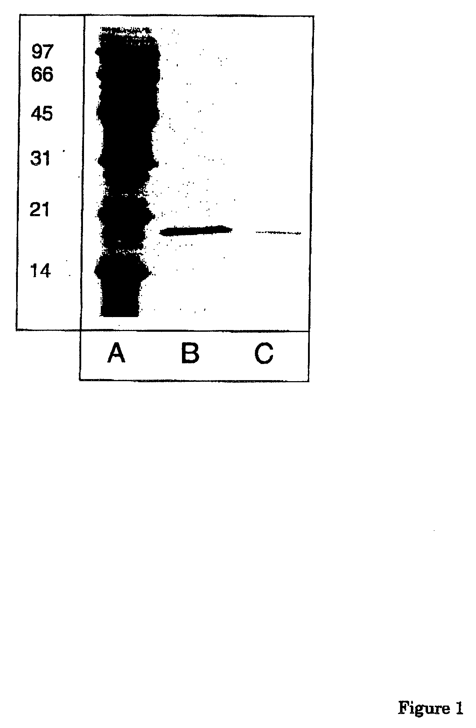

Since the expression level of His-MscL in *E. coli* was relatively low, based on the absence of a significant IPTG-inducible band on a SDS-PAGE, attention was focused on obtaining a high biomass during fermentation and a high yield after protein purification.
The His-tagged MscL could be purified to apparent homogeneity in a single step using nickel chelate affinity chromatography as shown by SDS-PAGE (Fig. 1, lane B). The yield of this eluted His-tagged MscL was ± 2 mg per Liter of cell-culture with an estimated purity of >98% based on analysis using SDS-PAGE and Coomassie Brilliant Blue staining.
The rate of excretion via MscL of small molecules is > 10 000 nmol/sec. x mg of cell protein, i.e. when the protein is in the open state. Since the expression level of mscL in wild-type bacteria is 4-10 functional units per cell and the MscL channel is a homopentamer of 15 000 Da, it can be concluded that the flux via a functional MscL channel is > $10^6$ x $s^{-1}$. This activity of MscL is such that on average 5 molecules of pentameric MscL per liposome with a diameter of 400 nm should suffice. Such a liposome contains approximately 1.67 x $10^6$ molecules of lipid; the molar ratio of lipid over MscL will thus be 0.67 x $10^5$. Consequently, 2 mg of MscL will yield 6 g of proteoliposomes.

Electrospray Ionization Mass Spectrometry of Detergent Solubilized MscL proteins.

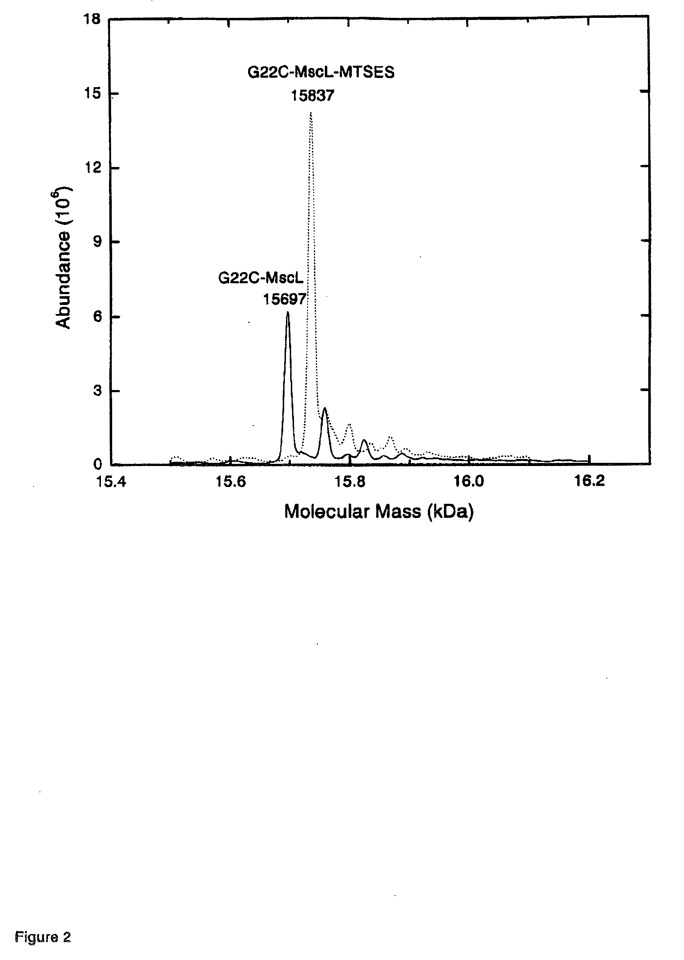

ESI-MS is an accurate and effective method to verify primary sequences of the 6His-MscL protein and the stoichiometry of conjugation reactions. Figure 2 shows the ESI-MS spectra of the G22C-MscL-6His and the MTSES conjugated G22C-MscL-6His samples.
Based on the deduced amino acids, the average molecular weight of G22C-MscL-6His is 15 826 Da. ESI-MS analysis of G22C-MscL-6His resulted in a molecular weight of 15 697 Da, which corresponds to the deduced molecular weight minus a methionine. This observation would be consistent with an excision of the N-terminal methionine as reported for many proteins expressed in *E. coli* [5]. ESI-MS analysis of the MTSES conjugated G22C-MscL-6His resulted in a molecular weight of 15 837 Da, which corresponds exactly with the calculated mass increase of the MTSES conjugation. ESI-MS analysis is routinely used to verify the average masses of MscL mutants and the products of conjugation reactions.

Membrane reconstitution into liposomes of different lipid compositions.

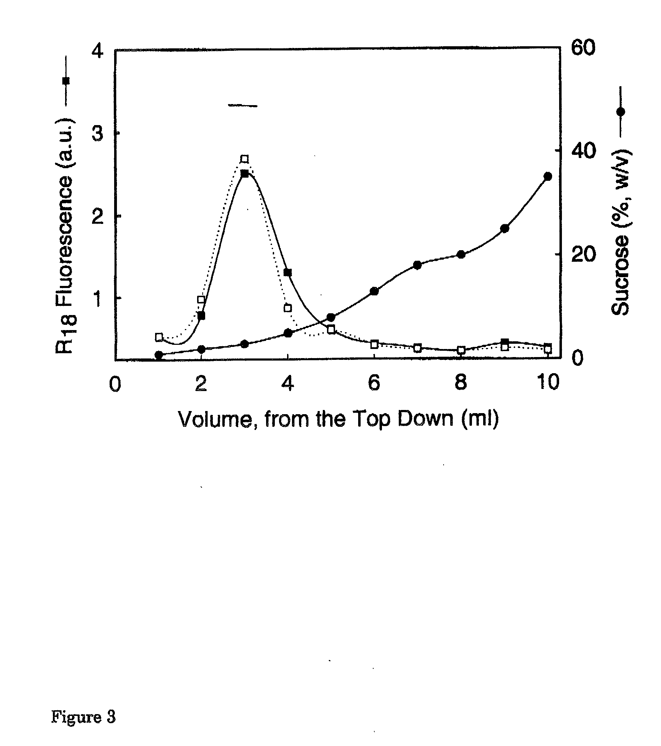

Purified detergent-solubilized MscL was reconstituted into preformed liposomes, which were titrated with low amounts of detergent. After removal of the detergent by adsorption onto polystyrene beads, proteoliposomes were formed. The proteoliposomes were characterized by equilibrium sedimentation on a sucrose gradient as shown in figure 3. All 6His-MscL protein detected by the Western blot (inset in Fig. 3) was associated with the lipid bilayer as detected by octadecylrhodamine-β-chloride ($R_{18}$) fluorescence.

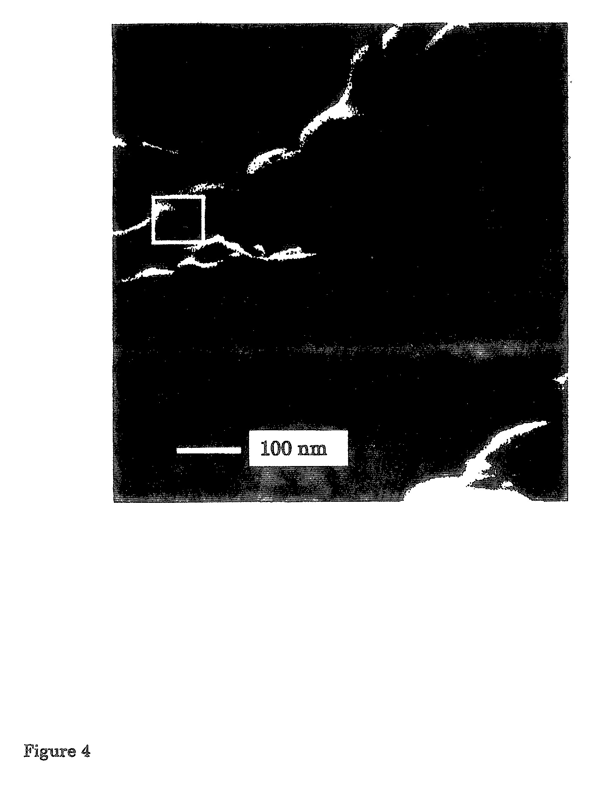

Association of the 6His-MscL protein with the liposomes does not necessarily mean the protein is inserted correctly into the lipd bilayer. Correctly inserted MscL protein should be trans-membrane, and show up as an intra-membrane vesicle (IMP) in a freeze-fracture image as shown in the white boxed area of figure 4.

The equilibrium sedimentation and freeze-fracture electron microscopy experiments provided structural evidence for the correct reconstitution of the 6His-MscL protein into lipid bilayers.

Electrophysiologic characterization of membrane-reconstituted MscL activity.

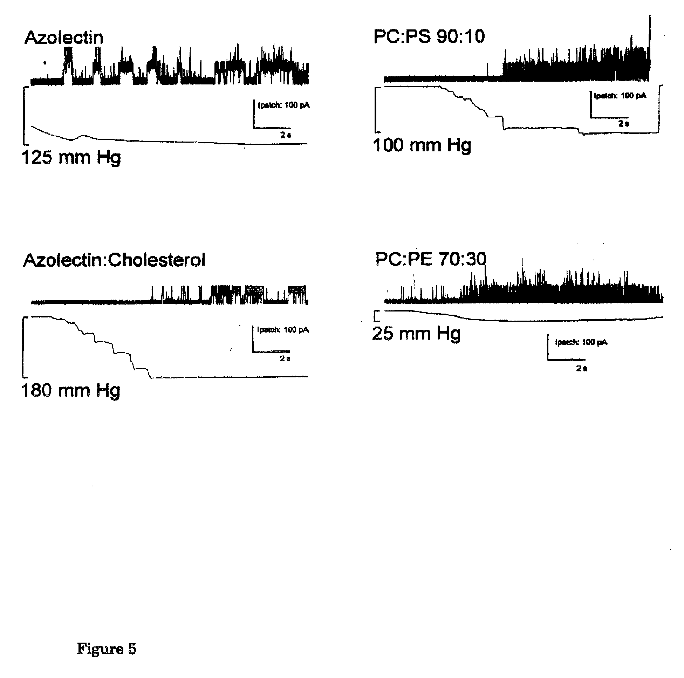

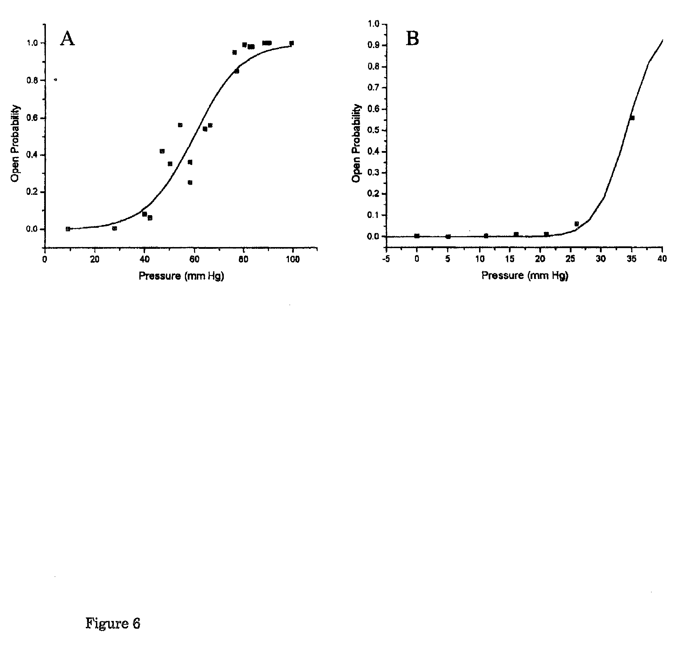

The purified protein reconstituted into phospholipid liposomes forms functional mechanosensitive channels, as seen from the traces in Fig. 5 at different pipette pressures (mechanical activation). The MscL open probability plotted against pressure can be fitted with a Boltzmann distribution (Fig. 6).

Interestingly, reconstituted MscL is active in the absence of negatively charged lipid headgroups (Fig. 5, PC:PE 70:30)). This is a very important finding since negatively charged headgroups prevent targeting to most target sites in the human body. Additionally, these experiments have shown for the first time that the pressure treshold is significantly effected by the lipid composition of the membrane reconstituted MscL channels (Fig. 6). This allows tailor making of the drug release profiles to the specific neads.

We have constructed several 6His-MscL mutants with altered gating properties, e.i.; mutants that are hypersensitive to membrane tension, and mutants with increased open probability at lower pH-values.

In vitro release profiles of a model drug.

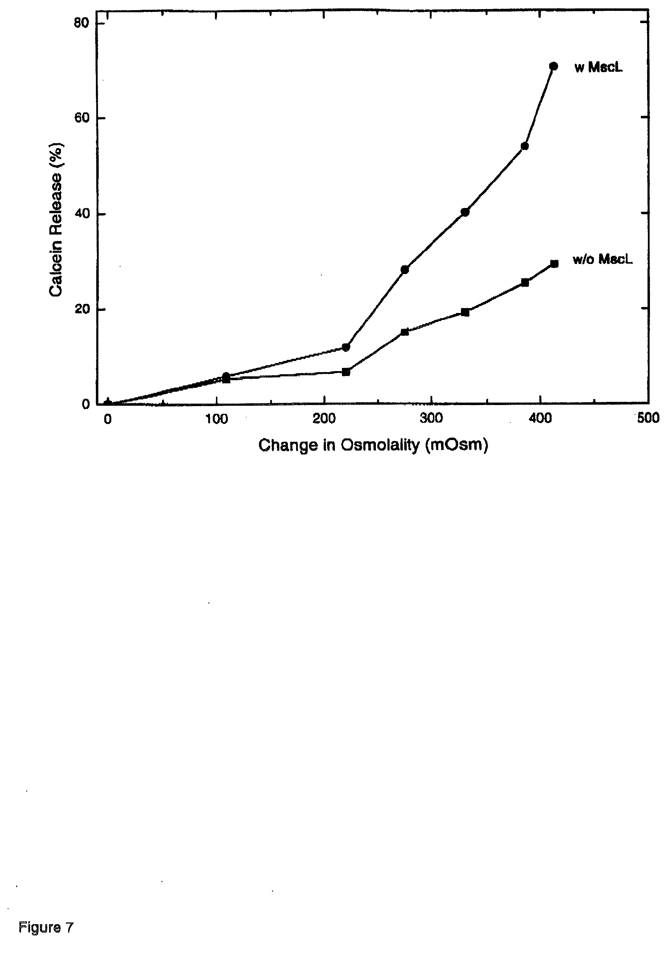

The fluorescence efflux-assay was developed to monitor the MscL-mediated release profiles.. Liposomes (DOPC:Chol, 60:40, m/m) with and without MscL-6His were subjected to an osmotic downshock, thereby effectively increasing the membrane tension, to monitor the calcein release. As shown in Fig. 7, less calcein remained in the liposomes containing MscL (closed circles) relative to the liposomes without MscL (closed squares) when change in osmolality was larger than 200 mOsm. These data demonstrate that, upon osmotic downshock, liposomes containing reconstituted MscL exhibit a greater efflux of calcein than liposomes without MscL. This MscL-mediated efflux is consistent with the electro-physiologic analysis, showing that the MscL is reconstituted into membranes of synthetic lipids while retaining its functional properties.

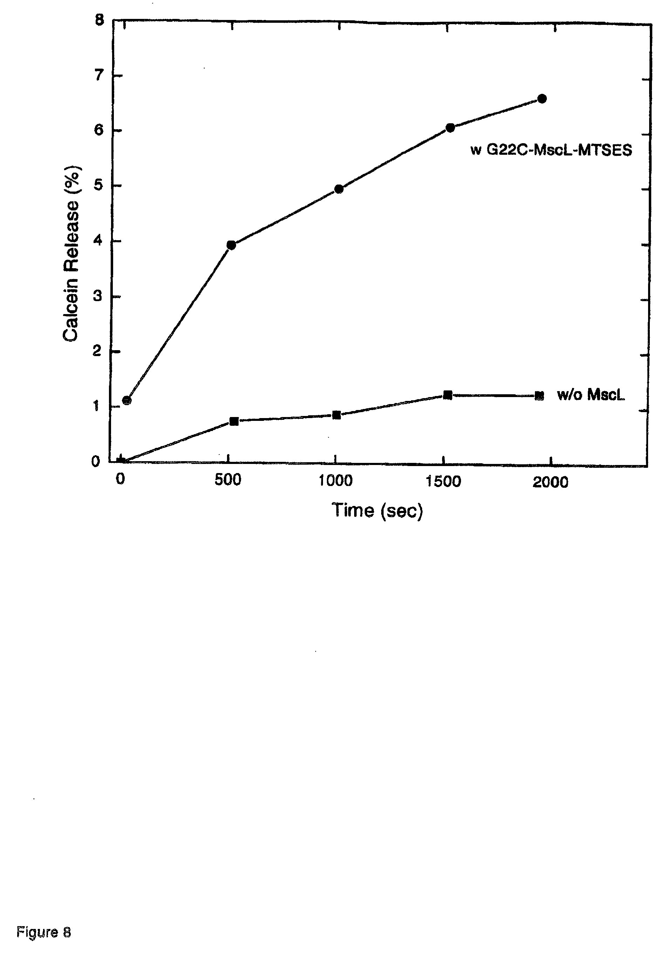

For controlled release of drugs at the target site, membrane tension may not be the most promising stimulus since little is known about osmotic differences in the human body. Several alternatives to activate the MscL channel at the target site are described in this patent. Introducing a charge through conjugation of MTSES to cysteine at position 22 serves as an example for channel activation under iso-osmotic conditions. ESI-MS analysis of the MTSES conjugated G22C-MscL-6His protein showed that all MscL monomers were conjugated to a stoichiometry of 1:1. The conjugated G22C-MscL-6His samples was subsequently reconstituted into liposomes as described above and calcein release was measured as shown in Fig. 8. These data demonstrate that, in the absence of an increased membrane tension, MscL exhibits drug release from drug laden synthetic liposomes. This patent includes MscL conjugates that will release drugs at the target site as a function of pH, light activation and specific interactions with target associated molecules.

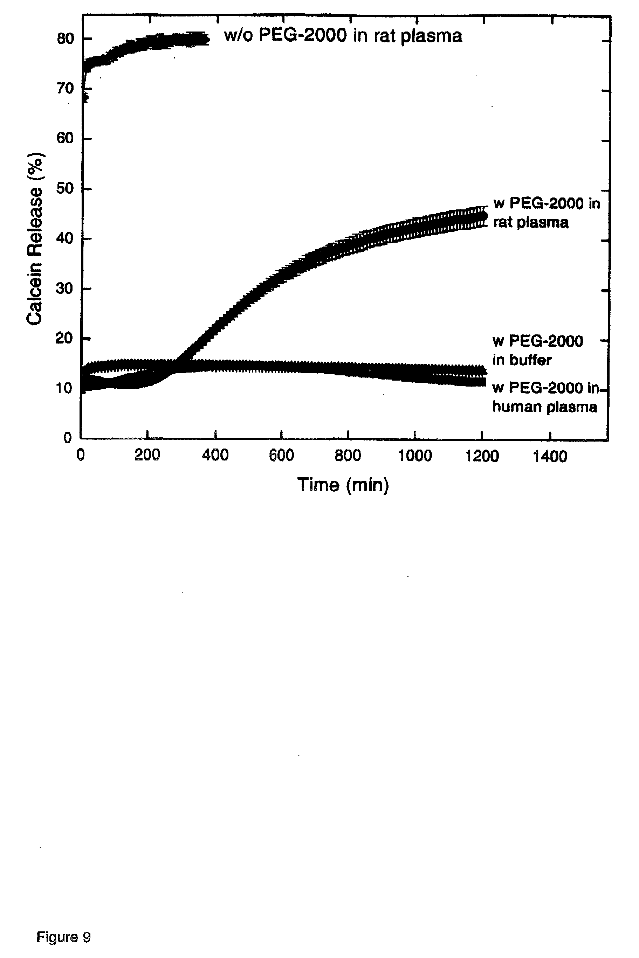

The integrity of liposomes consisting of phosphatidylcholine is seriously affected at first contact with a biological milieu following intravenous injection [15]. The integrity of liposomes (PC:Chol:DGPE-PEG, 55:40: 5, m/m/m) was studied as a function of the molecular mass of the PEG group attached to the DGPE lipid in the presence of rat and human plasma at 37 °C. Calcein release from only the liposomes without DGPE-PEG and with 5 mol% DGPE-PEG(2000) are shown in Fig. 9. These data show that addition of 5 mol% of DGPE-PEG(2000) significantly increase liposomal integrity in rat and human plasma up to several hours and will serve to prevent drug leakage during the traveling time to the target cells

FIGURE LEGENDS

Figure 1. SDS-PAGE stained with Coomassie Brilliant Blue (molecular weight markers indicated in kDa on the left of lane A, and purified detergent-solubilized MscL in lane B), and the Western blot (lane C).

Figure 2. Electrospray ionization mass spectrometry of G22C-MscL-6His and its MTSES conjugate. (solid line) Spectrum of G22C- MscL-6His. Not all peptides that are present in the sample are indicated in the spectrum. (broken line) Spectrum of MTSES conjugated G22C-MscL-6His. The masses are indicated at the peaks and show that all proteins are conjugated

Figure 3. Equilibrium centrifugation on sucrose gradients of proteoliposomes. 6His-MscL purified with Triton X-100 and incorporated in liposomes titrated with 4.0 mM Triton X-100 (Rsat; open squares) and liposomes titrated with 10.0 mM Triton X-100 (Rsol; closed squares). After centrifugation, the gradients were fractionated (0.5 mL) and assayed for the presence of lipids and protein. All protein, as determined by Western blotting as shown in the inset, is shown to be associated with the lipids, as determined by measuring fluorescence (AU) of $R_{18}$.

Figure 4. Freeze-fracture image of proteoliposome showing the MscL channel protein as a transmembrane vesicle (white box).

Figure 5. Patch-clamp recordings of channel activities at -20 mV from MscL reconstituted into liposomes of different lipid compositions as indicated in the figures. Pressure in the pipette, relative to atmospheric, is shown in the lower traces, and recording of the current through a patch of membrane excised from a blister is shown in the upper traces.

Figure 6. Pressure dependence of the MscL channel reconstituted in liposomes of different lipid composition. Open probability in the patch of a membrane with a lipid composition of PC: PS, 90:10 m/m (A) and PC: PE, 70:30, m/m (B) versus the applied pressure. Smooth curves are Boltzman fits.

Figure 7. Calcein efflux from liposomes with (closed circles) and without (closed squares) MscL as a function of a decrease in Osmolality of the external medium. A small volume (typically 20 µL) containing (proteo)liposomes in iso-osmotic buffer, is rapidly diluted with buffer of decreasing osmolality and calcein release was determined by dividing the fluorescence at 100 sec after dilution by the total fluorescence obtained after Triton X-100 lysis.

Figure 8. Calcein release under iso-osmotic condition mediated by conjugated G22C-MscL-6His. Calcein release of MTSES conjugated G22C-MscL-6His channel protein reconstituted into liposomes (PC: Chol, 60:40, m/m) (closed circles). Liposomes with the same lipid composition and sample treatment as above but without MscL (closed squares).

Figure 9. Effect of 5 mol% DGPE-PEG(2000) on the calcein release from liposomes (PC:Chol, 60:40, m/m). Calcein release from PC:Chol:DGPE-PEG(2000) liposomes in the presence of buffer (closed triangles), rat plasma (closed circles), human plasma (closed squares). Calcein release from liposomes without DGPE-PEG(2000) (closed diamond).

REFERENCES

1. Arkin, I.T., Sukharev, S.I., Blount, P., Kung, C., and Brünger A.T. (1998) *Biochim. Biophys. Acta* **1369,** 131-140.
2. Delcour, A.H., Martinac, B., Adler, J., and Kung, C. (1989) *Biophys. J.* **56,** 631-636.
3. Blount, P., Sukharev, S.I., Moe, P.C., Schroeder, M.J., Guy, H.R., and Kung, C. (1996) *EMBO J.* **15,** 4798-4805
4. Van Montfort, B.A., Cana, B., Duurkens, R., Godovac-Zimmermann, J., and Robillard, G.T. submitted
5. Hirel, A.U., Schmitter, M.J., Dessen, P., Fayat, G., and Blanquet, S. (1989) *Proc. Natl. Acad. Sci. U.S.A.* **86,** 8247-8251
6. Knol, J., Sjollema, and Poolman, B. (1998) *Biochemistry* **37,** 16410-16415
7. Friesen, R.H.E., Knol, J., Poolman, B. (2000) *J. Biol. Chem.* **43,** 33527-33535
8. Moe, P.C., Blount, P. and Kung, C. (1998) *Mol. Microbiol.* **28,** 583-592
9. Yoshimura, K., Batiza, A., Schroeder, M., Blount P., and Kung, C. (1999) *Biophys. J.* **77,** 1960-1972
10. Song, X., Perlstein, J., and Whitten, D.G. (1997) *J. Am. Chem. Soc.* **119,** 9144-9159
11. Blount, P., Sukharev, S.I., Schroeder, M.J., Nagle, S.K., and Kung, C. (1996) *Proc. Natl. Acad. Sci USA* **93,** 11652-11657
12. Mayer, L.D., Hope, M.J., and Cullis, P.R. (1986) *Biochim. Biophys. Acta* **858,** 161-168
13. Martinac, B., Adler, J., and Kung, C. (1990) *Nature* **348,** 261-263
14. Ou, X., Blount, P., Hoffman, R.J., and Kung C. (1998) *Proc. Natl. Acad. Sci. USA* **95,** 11471-11475
15. Damen J., Regts, J., and Scherphof G. (1981) *Biochim. Bioph. Acta* **665,** 538-545
16. Yoshimura, K, Batiza and Kung, C. (2001) Biophysical Journal (80) 2198-2206.

**Claims**

**1.** A method for delivering a small hydrophilic molecule to a cell, comprising loading a lipid vesicle with said small molecule and administering said vesicle to fluid that is in contact with said cell, said vesicle further comprising a proteinaceous channel that in the open state allows passage of said small molecule to the exterior of said vesicle, said lipid vesicle and/or said proteinaceous channel formulated such that said channel is in the open state in the vicinity of said cell.

**2.** A method according to claim 1, wherein vesicle is provided to an animal or a human.

3. A method according to claim 1 or claim 2, wherein said vesicle is provided to a human.

4. A method according to any one of claims 1-3, wherein said proteinaceous channel comprises an solute channel.

5. A method according to any one of claims 1-4, wherein said proteinaceous channel comprises a mechanosensitive channel.

6. A method according to claim 5, wherein said proteinaceous channel comprises a mechanosensitive channel of large conductance (MscL) or a functional equivalent thereof.

7. A method according to any one of claims 1-6, wherein said lipid vesicle is formulated to allow preferential opening of said channel near said cell.

8. A method according to any one of claims 1-7, wherein said lipid vesicle comprises positively and/ or neutrally charged lipids.

9. A method according to claim 8, wherein said lipid vesicle consists predominantly of positively and/or neutrally charged lipids.

10. A method according to anyone of claims 1-9, wherein said MscL is a mutant MscL.

11. A method according to claim 10, wherein said mutant allows preferred release of said small molecule in the vicinity of said cell.

12. A method according to any one of claims 1-11, wherein said small molecule comprises a peptide.

13. A method according to any one of claims 1-12, wherein said small molecule comprises a diameter smaller than 60 Å.

14. A method according to claim 13, wherein said small molecule comprises a diameter smaller than 40 Å.

15. A method according to any one of claims 1-14, wherein activation of said channel is triggered upon the availability of a signal.

16. A method according to claim 15, wherein said signal comprises light.

17. A method according to claim 15 or claim 16 wherein said signal comprises an altered pH and/or temperature.

18. A method according to claim 17, wherein said pH is equal or less then 6.5.

19. A composition comprising a lipid vesicle comprising a proteinaceous channel and a small hydrophilic molecule, wherein said lipid vesicle and/or said proteinaceous channel is formulated such that said proteinaceous channel is in the open state in the vicinity of a target cell.

20. A composition according to claim 19, wherein said proteinaceous channel comprises an MscL or functional part, derivative and/or analogue thereof.

21. A composition comprising a lipid vesicle comprising an MscL or functional part, derivative and/or analogue thereof, wherein said composition is formulated and prepared for use in a human.

22. A composition according to any one of claims 19-21, wherein said MscL is a mutant MscL or a functional part, derivative and/or analogue thereof.

23. A composition according to any one of claims 19-22, wherein said vesicle comprises an asymmetrical bilayer.

24. A composition according to any one of claims 19-23, wherein said vesicle comprises a light sensitive lipid and/or MscL.

25. A composition according to any of claims 19-24, wherein activation of channel is triggered by light, pH or temperature.

26. A composition according to any one of claims 19-25, wherein a signal required for activation is provided through an intermediate.

27. A composition according to any one of claims 19-26, wherein said lipid vesicle comprises a neutral lipid.

28. A composition according to any one of claims 19-27, wherein said lipid vesicle comprises a positively charged lipid.

29. A composition according to claim 27 or claim 28, wherein said lipid vesicle does essentially not comprise negatively charged lipid.

30. A composition according to any one of claims 19-29, further comprising a small hydrophilic molecule capable of passing through an activated MscL.

31. A composition according to any one of claims 19-30, further comprising a non-channel protein.

32. A composition according to claim 31, wherein said protein is a binding molecule capable of binding to a binding partner on said cell thereby enabling at least a prolonged stay of said vesicle near said cell.

**33.** Use of a composition according to any one of claims 19-32, for the preparation of a medicament.

**34.** Use of a composition according to any one of claims 19-33, for the preparation of a medicament wherein said small molecule is intended to be delivered to the outside of said cell.

**35.** Use of a lipid vesicle comprising an MscL for controlling delivery of a small hydrophilic molecule to a target tissue in a body.

**36.** A method for generating a vehicle for delivery of a small hydrophilic molecule to a cell, said method comprising generating in an aqueous fluid, a lipid vesicle comprising a proteinaceous channel, said vehicle formulated such that said proteinaceous channel is in the open state in the vicinity of said cell.

**37.** A method according to claim 36, wherein said proteinaceous channel assumes said open state upon entering the vicinity of said cell.

**38.** A method according to claim 37, wherein said lipid vesicle further comprises said small molecule.

Figure 1

Figure 2

Figure 3

Figure 4

Azolectin

PC:PS 90:10

Ipatch: 100 pA

2 s

125 mm Hg

100 mm Hg

Azolectin:Cholesterol

PC:PE 70:30

Ipatch: 100 pA

2 s

180 mm Hg

25 mm Hg

Ipatch: 100 pA

2 s

Figure 5

Figure 6

Figure 7

Figure 8

18

Figure 9

## PARTIAL EUROPEAN SEARCH REPORT

**European Patent Office**

which under Rule 45 of the European Patent Convention shall be considered, for the purposes of subsequent proceedings, as the European search report

**Application Number**

EP 01 20 2401

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 20252 A (CLARKE DAVID JOHN ;UNIV MANCHESTER (GB); HARRISON MICHAEL HENRY (G) 29 April 1999 (1999-04-29)<br><br>* page 4, paragraph 2 *<br>* page 5, paragraph 2 - page 9, paragraph 1 *<br>* page 25, last paragraph - page 29, paragraph 3 *<br>* example 2.1 *<br>* claims 1-6,11,23-31 * | 1-4,7-9, 12-15, 17-22, 24-27, 29-34, 36-38 | A61K9/127 |
| X | US 5 820 879 A (FERNANDEZ JULIO M ET AL) 13 October 1998 (1998-10-13)<br><br>* column 2, line 14 - line 24 *<br>* column 19, line 18 - column 32, line 21; figure 16 *<br>* claims 1-25 * | 1-4,7-9, 12-15, 19-24, 26-34, 36-38 | |
| | -/-- | | **TECHNICAL FIELDS SEARCHED (Int.Cl.7)**<br><br>A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search :

Although claims 1-18 and 35 are directed to a method of treatment of the human/animal body (Article 52(4) EPC), the search has been carried out and based on the alleged effects of the composition.

| Place of search | Date of completion of the search | Examiner |
|---|---|---|
| THE HAGUE | 22 November 2001 | Epskamp, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**PARTIAL EUROPEAN SEARCH REPORT**

Application Number

EP 01 20 2401

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | SIMOES S ET AL.: "Enhancement of cationic liposome-mediated gene delivery by transferrin and fusogenic peptides" PROCEEDINGS OF THE INTERNATIONAL SYMPOSIUM ON CONTROLLED RELEASE OF BIOACTIVE MATERIALS, vol. 24, 15 - 19 June 1997, pages 659-660, XP002098090 ISSN: 1022-0178 * the whole document * | 1-4,7-9, 12-15, 19-22, 24,26, 27,29, 30,33, 34,36-38 | |
| X | HÄSE CC ET AL.: "Purification and functional reconstitution of the recombinant large mechanosensitive ion channel (MscL) of Escherichia coli." JOURNAL OF BIOLOGICAL CHEMISTRY, vol. 270, no. 31, 1995, pages 18329-18334, XP002131093 ISSN: 0021-9258 * abstract * * page 18; figure 1 * | 19-22, 24,26, 27,29, 30,33, 34,36-38 | TECHNICAL FIELDS SEARCHED (Int.Cl.7) |
| X | HÄSE CC ET AL.: "Molecular dissection of the large mechanosensitive ion channel (MscL) of E. coli: Mutants with altered channel gating and pressure sensitivity." JOURNAL OF MEMBRANE BIOLOGY, vol. 157, no. 1, 1997, pages 17-25, XP002183679 ISSN: 0022-2631 * abstract * * page 18330 "Reconstitution of recombinant proteins in artificial liposomes" * | 19-22, 24,26, 27, 29-34, 36-38 | |

-/--

EPO FORM 1503 03.82 (P04C10)

| | European Patent Office | **PARTIAL EUROPEAN SEARCH REPORT** | Application Number EP 01 20 2401 |
|---|---|---|---|

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | MARTINAC B: "Mechanosensitive channels in prokaryotes." CELLULAR PHYSIOLOGY AND BIOCHEMISTRY, vol. 11, no. 2, 2001, pages 61-76, XP002183680 ISSN: 1015-8987 * abstract * * page 64 - page 70, right-hand column, paragraph 2 * | 1-38 | |
| | | | TECHNICAL FIELDS SEARCHED (Int.Cl.7) |

EPO FORM 1503 03.82 (P04C10)

**ANNEX TO THE EUROPEAN SEARCH REPORT
ON EUROPEAN PATENT APPLICATION NO.**                     EP 01 20 2401

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2001

| Patent document cited in search report | Publication date | Patent family member(s) | | Publication date |
|---|---|---|---|---|
| WO 9920252 A | 29-04-1999 | AU | 9547398 A | 10-05-1999 |
| | | EP | 1023047 A1 | 02-08-2000 |
| | | WO | 9920252 A1 | 29-04-1999 |
| | | NO | 20001976 A | 15-06-2000 |
| US 5820879 A | 13-10-1998 | US | 5654006 A | 05-08-1997 |
| | | US | 5811124 A | 22-09-1998 |
| | | AT | 162397 T | 15-02-1998 |
| | | AU | 676543 B2 | 13-03-1997 |
| | | AU | 6247994 A | 29-08-1994 |
| | | CA | 2155648 A1 | 18-08-1994 |
| | | DE | 69408122 D1 | 26-02-1998 |
| | | DE | 69408122 T2 | 03-09-1998 |
| | | DK | 684812 T3 | 21-09-1998 |
| | | EP | 0684812 A1 | 06-12-1995 |
| | | ES | 2114184 T3 | 16-05-1998 |
| | | JP | 8509956 T | 22-10-1996 |
| | | WO | 9417786 A1 | 18-08-1994 |
| | | US | 5753261 A | 19-05-1998 |

EPO FORM P0459

For more details about this annex : see Official Journal of the European Patent Office, No. 12/82